(19) **Europäisches Patentamt**

**European Patent Office**

**Office européen des brevets**

(11) **EP 1 075 858 A1**

(12) **EUROPEAN PATENT APPLICATION**
published in accordance with Art. 158(3) EPC

(43) Date of publication:
**14.02.2001 Bulletin 2001/07**

(51) Int. Cl.[7]: **A63B 22/06**, A63B 23/04,
A63B 71/06, A61B 5/05

(21) Application number: **99917186.1**

(22) Date of filing: **27.04.1999**

(86) International application number:
**PCT/JP99/02245**

(87) International publication number:
**WO 99/55428 (04.11.1999 Gazette 1999/44)**

(84) Designated Contracting States:
**AT BE CH CY DE DK ES FI FR GB GR IE IT LI LU MC NL PT SE**

(30) Priority: **28.04.1998 JP 11977498**
**20.11.1998 JP 33130898**

(71) Applicant: **Yaman Ltd.**
**Tokyo 135-0045 (JP)**

(72) Inventors:
• **YAMAZAKI, Iwao**
**Yaman Ltd.**
**Koto-ku, Tokyo 135-0045 (JP)**

• **YAMAZAKI, Kimiyo**
**Yaman Ltd.**
**Koto-ku, Tokyo 135-0045 (JP)**

(74) Representative:
**Newstead, Michael John et al**
**Page Hargrave**
**Southgate**
**Whitefriars**
**Lewins Mead**
**Bristol BS1 2NT (GB)**

(54) **DEVICE FOR AUTOMATICALLY ADJUSTING LOAD ON AEROBIC EXERCISE MACHINE**

(57)    Disclosed is an automatic load control for a load-variable aerobics machine, which permits determination as to how much the machine is to be loaded, how often and how long the so loaded machine is to be used to reach a target physical shape, and permits the automatic controlling of the machine to follow a given aerobics program while the machine is being used.

It comprises a bodily impedance measuring means, data input means for inputting individual data pertaining to sex, age, height and weight, means for determining the instantaneous fat rate from the body impedance and the individual data; means for determining the current physical shape from the body-weight and bodily fat rate; means for setting a desired physical shape as a target; means for determining the number of times of exercise and setting the so determined exercise amount for a set exercise amount value; means for comparing the current physical shape with the target physical shape to determine the amount of exercise required for reducing the difference therebetween; means for determining on the basis of the required amount of exercise and the set exercise amount value an exercise program describing how much the load-variable aerobics machine is to be loaded, and how long the so loaded aerobics machine is to be used; and means for changing the amount of load on the load-variable aerobics machine to be in conformity with the load given in the exercise program, whereby the loading of the aerobics machine may be controlled to consume the amount of exercise required for reaching the target physical shape according to the exercise program.

EP 1 075 858 A1

## Description

### Technical Field

The present invention relates to an aerobics machine which exercise one's arms and legs for the purpose of supplying his body with oxygen and burning his bodily fat such as a treadmill for walking or jogging, a bicycle ergometer for pedalling or a stair climber.

### Background Art

A human body is composed of bodily fat and non-fat texture such as muscle and bones. The body-weight is equal to the weight of bodily fat plus the weight of non-fat texture.

The body-weight is a measure representing corpulence. One's physical or bodily shape can be classified into the pyknic type, the muscular type and the intermediate type of different degrees in terms of how much the bodily fat is in the body-weight.

Persons of pyknic type are significantly different from persons of muscular type not only in health condition but also in exercising modes and capabilities. They are different in appearance, too. As is well realized, persons having same body-weight are often different in physical shape. The physical shape, therefore, is likely to show one's exercising capability, health condition, appearance and other physical or biological characteristics. Such physical shape can be controlled by controlling the bodily fat rate relative to the body-weight by taking bodily exercises.

What is aimed at by using an aerobics machine is to strengthen one's heart and lungs for increasing his endurance, and at the same time, remove extra amount of fat and body-weight, thereby giving him a fatless or muscled good shape.

To do this it is important that one has a desired physical shape as a target in mind, and that he realizes how much heavy a bodily exercise he must take, how often and how long he must continue such a bodily exercise to reach the target physical shape. Having a physical beauty in mind may be better in promotion in aerobics compared to the target fat removal given in numerical figure.

In view of the above one object of the present invention is to provide an automatic load control for a load-variable aerobics machine, which control permits a person to have a desired physical shape in mind, and realize how much and how long he takes a bodily exercise according to an exercise program which can be determined automatically on the basis of individual physical factors, thereby permitting him to continue such a bodily exercise in a most efficient way while automatically controlling the loading of the aerobics machine according to the exercise program, thus preventing him from being troubled or disturbed by manually controlling the loading of the aerobics machine.

### Disclosure of Invention

To attain this object an automatic load control for a load-variable aerobics machine according to one aspect of the present invention comprises: a bodily impedance measuring means having electrodes to be applied to a human body for determining the impedance of the human body; data input means for inputting individual data pertaining to sex, age, height and body-weight; means for determining the instantaneous fat rate from the body impedance and the individual data; means for determining the current physical shape from the body-weight and bodily fat rate; means for setting a desired physical shape as a target; means for determining the number of times of exercise and setting the so determined exercise amount for a set exercise amount value; means for comparing the current physical shape with the target physical shape to determine the amount of exercise required for reducing the difference therebetween; means for determining on the basis of the required amount of exercise and the set exercise amount value an exercise program describing how much the load-variable aerobics machine is to be loaded, and how long the so loaded aerobics machine is to be used; and means for changing the amount of load on the load-variable aerobics machine to be in conformity with the load given in the exercise program, whereby the loading of the aerobics machine may be controlled to consume the amount of exercise required for reaching the target physical shape.

An automatic load control for a load-variable aerobics machine according to another aspect of the present invention comprises: a bodily impedance measuring means having electrodes to be applied to a human body for determining the impedance of the human body; data input means for inputting individual data pertaining to sex, age, height and body-weight; means for determining the instantaneous fat rate from the bodily impedance and the individual data; means for making a decision of the current physical shape from the bodily fat and the body-weight; means for setting a desired physical shape for a target; means for determining the number of times of exercise and for setting the so determined number of times of exercise as a target; means for comparing the current physical shape with the target physical shape to determine the amount of exercise required for reducing the difference therebetween; means for determining on the basis of the required exercise amount and the target exercise amount an exercise program describing a target heartbeat rate and a length of period involved for using the aerobics machine; means for measuring the heartbeat rate during exercise; and means for changing the amount of load on the load-variable aerobics machine to permit the heartbeat rate measured during exercise to be equal to the target heartbeat rate, whereby the loading of the aerobics machine may be controlled to consume the amount of exercise required for reaching the target physical shape according to the exercise program.

**Brief Description of Drawings**

Fig.1 shows diagrammatically a treadmill for walking or jogging;
Fig.2 shows diagrammatically a bicycle ergometer for pedalling;
Fig.3 shows diagrammatically a stair climber;

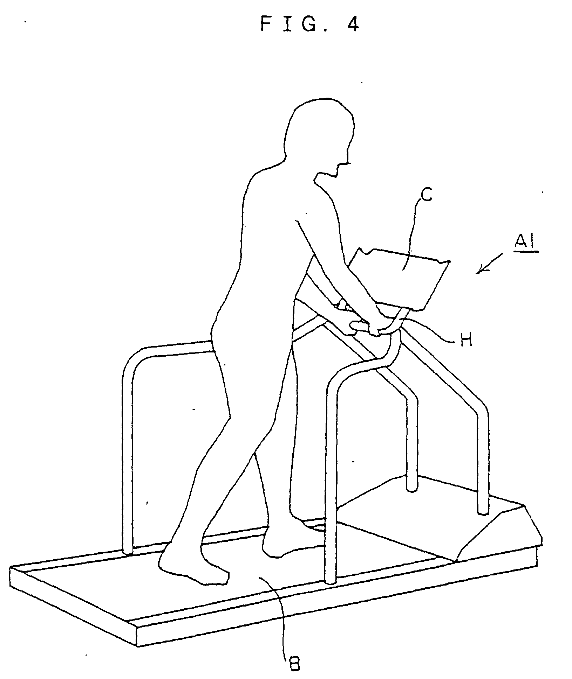

Fig.4 shows diagrammatically the manner in which a person uses a treadmill while measuring the bodily fat rate;
Fig.5 is a block diagram of an automatic load control according to the present invention;
Fig.6 shows a fragment of the grip rod in the automatic load control;
Fig.7 is a block diagram of a bodily impedance measuring circuit in the automatic load control;
Fig.8 is a physical shape diagram showing different physical shapes given by the physical shape determining means; and
Fig.9 shows how the current physical shape is given, and how the desired physical shape is given in the physical shape diagram.

**Best Mode of Reducing the Invention into Practice**

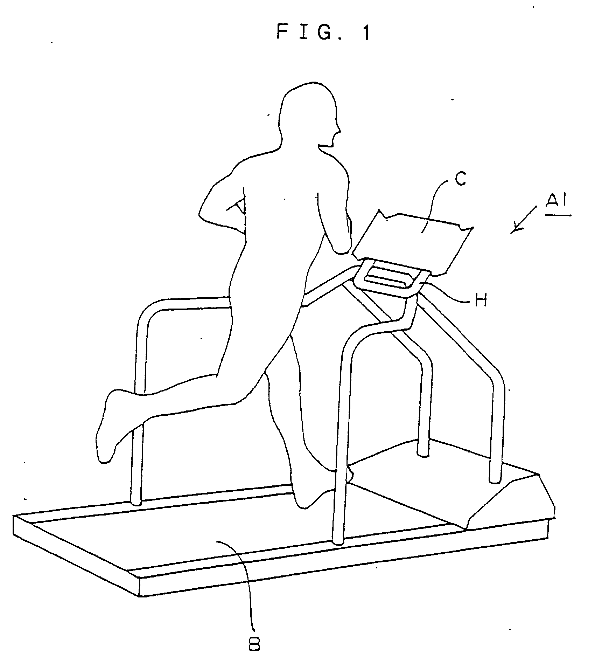

Figs.1 to 3 show diagrammatically different types of load-variable aerobics machines which can be equipped with an automatic load control according to the present invention.

Referring to Fig.1, a treadmill A1 permits a person to take a jogging or walking exercise by walking or running on its running belt B.

As shown in the drawing, the machine A1 has a colour liquid crystal display C mounted on its front side, and it is responsive to finger touch for showing the loading condition, the gradient of the running belt B, the heartbeat rate and other variables.

The loading can be controlled by changing the gradient of the running belt B and the braking torque applied to the belt shafts.

The bodily impedance and the heartbeat rate can be determined while gripping the crossbar, which is fixed to the lower side of the colour liquid crystal display C.

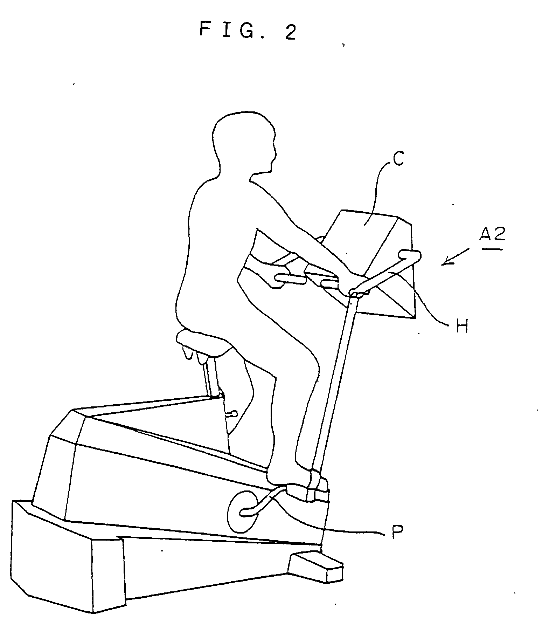

Referring to Fig.2, a bicycle ergometer A2 permits a person to take a pedalling exercise by using its pedals P.

As shown in the drawing, the machine A2 has a colour liquid crystal display C mounted on its front side, and it is responsive to finger touch for showing the loading condition, the running speed, the running distance, the heartbeat rate and other variables.

The loading can be controlled by changing the resistance to pedalling, which is caused by the friction of the pedals P, and by the braking torque applied to the pedal shafts.

The bodily impedance and the heartbeat rate can be determined while gripping the crossbar, which is fixed to the lower side of the colour liquid crystal display C.

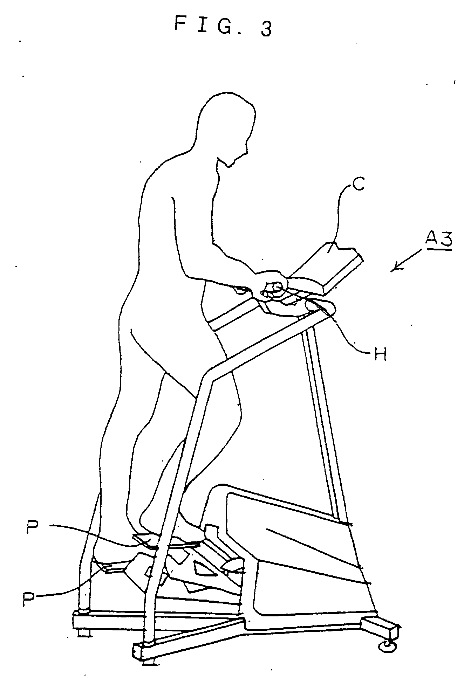

Referring to Fig.3, a stair climber A3 permits a person to take a stair climbing exercise by alternately pushing down its pedals P with his feet.

As shown in the drawing, the machine A3 has a colour liquid crystal display C mounted on its front side, and it is responsive to finger touch for showing the loading condition, the climbing speed, the number of steps the person has climbed, the heartbeat rate and other variables.

The loading can be controlled by changing the pedal weight and the braking torque to be applied to the pedal shafts.

The bodily impedance and the heartbeat rate can be determined while gripping the crossbar, which is fixed to the lower side of the colour liquid crystal display C.

One example of loading control comprises a shaft having a disk integrally connected thereto and an electromagnet closely adjacent to the disc. As a person is taking a bodily exercise, the shaft rotates, and eddy current is caused by the magnetic flux passing through the disc to cause a braking force to be applied to the disc. The loading can be controlled by controlling the electric current flowing in the electromagnet, thereby controlling the amount of braking effect.

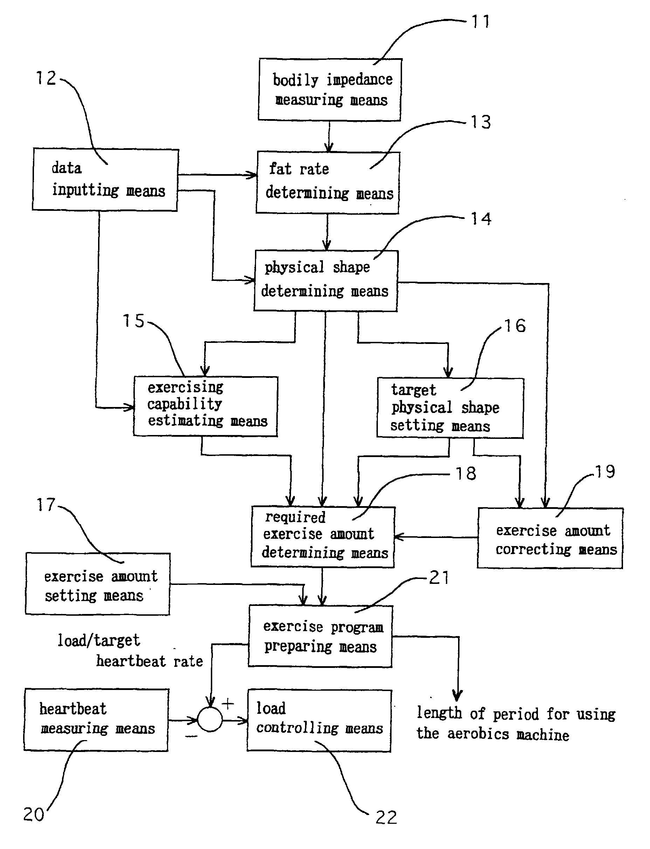

Referring to Fig.5, an automatic load control according to one embodiment of the present invention comprises: a bodily impedance measuring means 11 having electrodes in the form of grip crossbar H to be applied to a human body for determining the impedance of the human body; data input means 12 for inputting individual data pertaining to sex, age, height and body-weight with the aid of the colour liquid crystal display C; means 13 for determining the instantaneous fat rate from the bodily impedance and the individual data; means 14 for making a decision as to which physical shape of 29 different figures classified into 9 groups one's physical shape belongs to, judging from the bodily fat relative to the body-weight; means 15 for estimating one's capability of taking a bodily exercise from the non-fat texture amount (obtained by subtracting the bodily fat weight from one's body-weight, such as muscle and bones' weight), means 16 for selecting one of 29 physical shapes classified into 9 groups with reference to the current physical shape determined by the means 14, and for setting the desired physical shape as a target; means 17 for determining the number of times of exercise in terms of the period and the frequency of exercise per unit period required for reaching the target physical shape and for setting the so determined exercise amount for a set exercise amount value; means 18 for comparing the current physical shape with the target physical shape to determine the amount of exercise (the product of load and time) each time of exercise required for reducing the difference therebetween; means 19 for comparing the current physical shape with the target physical shape to renew the amount of exercise on the basis of the difference therebetween; means 20 for measuring the heartbeat rate through the agency of the electrodes of the grip crossbar H; means 21 for determining on the basis of the required amount of exercise and the set exercise amount value an exercise program describing the target heartbeat rate and the length of period for using the aerobics machine; and means 22 for changing the amount of load on the load-variable aerobics machine to permit the heartbeat rate measured by the means 20 to be equal to the target heartbeat rate.

While taking a bodily exercise, one's body-weight can be determined for instance, by using a strain gauge, which may be installed in the aerobics machine.

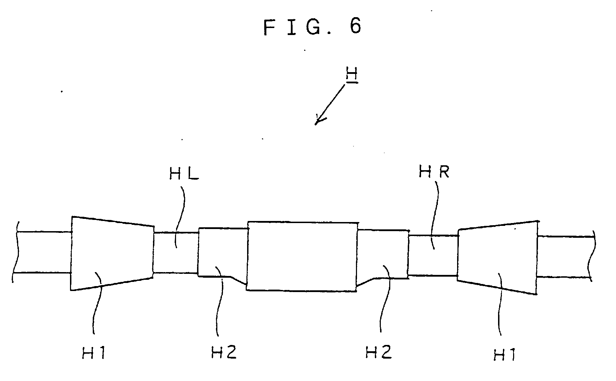

Referring to Fig.6, the grip crossbar H comprises an insulating rod having left and right grips HL and HR integrally connected to its opposite ends, and these grips HL and HR have feeding electrodes H1 and H1 and detecting electrodes H2 and H2 applied therearound. The feeding electrodes H1 are separated from the detecting electrodes H2.

These electrodes can be placed at different positions, for examples, the surrounding surface of the colour liquid crystal display C.

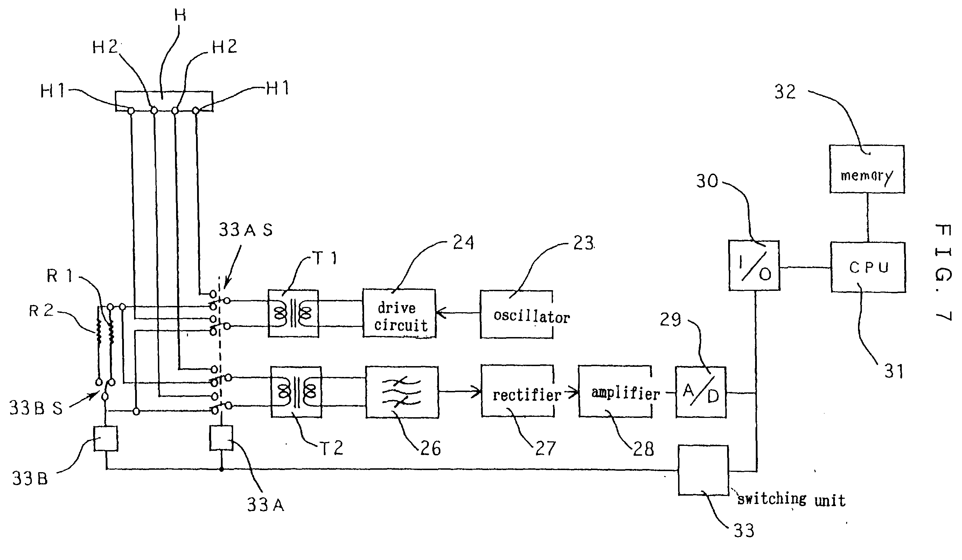

Fig.7 is a block diagram of a measuring circuit of the bodily impedance measuring means 11. As shown in the drawing, an oscillator 23 generates sinusoidal voltage at 50 kHz, which is fed to the feeding electrodes H1 and H1 via a drive circuit 24, a transformer T1 and a switch 33As. When a person holds the left and right grips HL and HR in his hands, an ac voltage appears between the detecting electrodes H2 and H2. The ac voltage is converted to a corresponding dc voltage after passing through the switch 33As, a transformer T2, a band filter 26, a rectifier 27 and an amplifier 28, and then, the dc voltage is directed to a CPU 31 via an A/D converter 29 and an I/O interface 30 after being shaped, level-controlled and offset-adjusted. The CPU 31 has a memory 32 associated therewith.

In an attempt to reduce a measurement error which would be caused by the time variability and temperature characteristics of some circuit components the measuring circuit must be calibrated in terms of its input-to-output characteristics prior to measurement of bodily impedance. Specifically the calibration can be effected by using the following equation:

$$Z = k\,V + C0,$$

where Z stands for bodily impedance; V stands for the voltage appearing between the detecting electrodes H2; k stands for a proportionality factor; and C0 stands for a fixed constant.

The same ac voltage as used in measuring bodily impedance Z is applied across two resistances R1 and R2 of known values to determine the voltages appearing across these known resistances R1 and R2. By substituting the known values of R1 and R2 and the so determined voltages for Z and V in two equations two constants k and C0 can be determined.

To do this the CPU 31 directs a control signal to the switching control 33A via the I/O interface 30 and a switching unit 33 to put the resistances R1 in the measuring circuit. Then, the CPU 31 directs another control signal to the switching control 33B via the I/O interface 30 and the switching unit 33 to put the resistance R2 in the measuring circuit.

The bodily impedance measuring means 11 can determine a bodily impedance according to the following equation 1:

Bodily Impedance = A x Gradient of the Curve x Measured Value + Ordinates Intersection of the Curve

| A = 0.792 | crossbar | male |
| 0.825 | crossbar | female |
| 0.893 | electrode | male |
| 0.95 | electrode | female |

$$\text{Gradient} = (810 - 310)/(\text{Upper Measured Value} - \text{Lower Measured Value})$$

$$\text{Intersection} = 810 - \text{Gradient} \times \text{Upper Measured Value}$$

The bodily fat rate measuring means 13 can determine a bodily fat rate according to the following equation 2:

Females:

$$\text{Bodily Fat Rate} = (1 - \text{Non-Fat Texture}/ \text{Weight}) \times 100$$

$$\text{Non-Fat Texture} = 0.6483 \times (\text{Height} \times \text{Height})/\text{Impedance} + B \times \text{Weight} + 5.091$$

| B = 0.1699 | Height: 150 cm or taller | |
| 0.12 | Height: below 150 cm | Upper Limit of Ideal Weight + 2.5 or more |
| 0.13 | | Upper Limit of Ideal Weight + 2.0 or more |
| 0.14 | | Upper Limit of Ideal Weight + 1.5 or more |
| 0.15 | | Upper Limit of Ideal Weight + 1.0 or more |
| 0.16 | | above Upper Limit of Ideal Weight |
| 0.1699 | | below Upper Limit of Ideal Weight |

$$\text{Upper Limit of Ideal Weight} = (\text{Height} - 100) \times 0.9 \times 1.1$$

Males:

$$\text{Bodily Fat Rate} = (4.95/\text{Bodily Density} - 4.5) \times 100$$

$$\text{Bodily Density} = 1.1554 - (0.0841 \times \text{Weight} \times \text{Impedance})/(\text{Height} \times \text{Height})$$

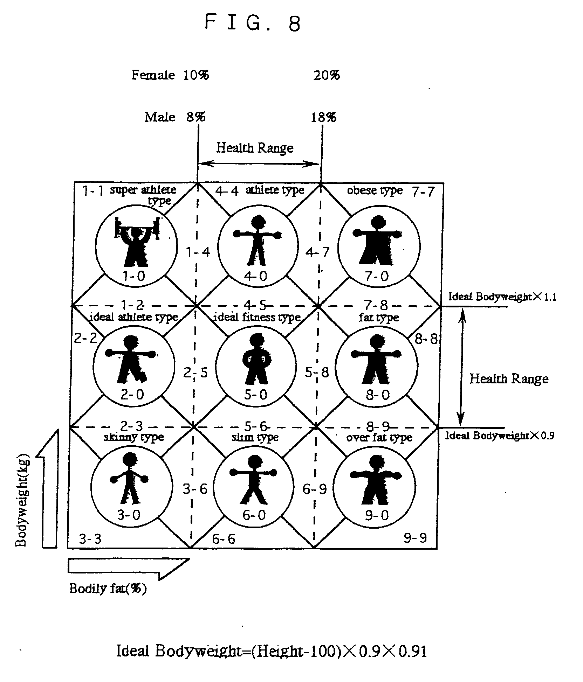

Referring to Fig.8, the means 14 shows on the colour liquid crystal display a physical shape diagram having different physical shapes each determined from the bodily fat rate relative to the body-weight. It is a 3x3 lattice representing body-weight on its ordinate and bodily fat on its abscissae. Also, it has indications of "Good Health" on its intermediate section, dividing the ordinate and abscissae in three levels in terms of body-weight and bodily fat, respectively.
The body-weight range for "Good Health" is from 0.9 x ideal body-weight to 1.1 x ideal weight. The bodily fat range for "Good Health" is from 10% to 20% (female) and from 8% to 18% (male).

The nine squares show the physical shapes of "SUPER ATHLETIC TYPE", "ATHLETIC TYPE", "PYKNIC TYPE", "IDEAL ATHLETIC TYPE", "IDEAL HEALTH TYPE", "EXCESSIVE FAT TYPE", "EXTRA SLENDER TYPE", "SLENDER TYPE", and "SUPER EXCESSIVE FAT TYPE". The ordinate and abscissae are subdivided to provide twenty nine physical shapes.

The ideal body-weight is given by:

$$\text{Ideal Body-Weight (Kg)} = (\text{Height (cm)} - 100) \times 0.9 \times 0.91$$

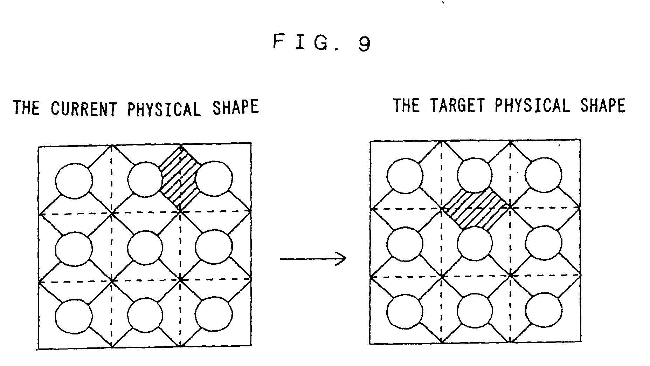

The current physical shape is indicated by changing the brightness of a selected square or by flickering in the selected square in the colour liquid crystal display, as seen from Fig.9.

Means 15 estimates one's capability of taking a bodily exercise, thus permitting the hardness of exercise appropriate for individuals to be determined on the basis of the so estimated exercising capability, so that the safety may be assured in taking a bodily exercise, still causing a maximum exercise effect.

Usually such one's capability is estimated on the basis of maximum oxygen-intake amount, but in the measurement discussed here it is estimated in terms of non-fat texture (determined by subtracting bodily fat from body-weight), which is thought of as a measure of performance.

It is assumed that the hardness of bodily exercise which can be regarded as a measure of effectively burning the bodily fat is within the range from 60% to 90% of the maximum heart beat. The maximum heart beat can be given as follows:

$$\text{Maximum Heartbeat} = 220 - \text{Age}$$

Therefore, the age is one of the factors which are thought of in estimating one's exercising capability, and the sex is another factor.

Means 16 permits the person to select a desired physical shape among the twenty nine physical shapes classified into nine groups given in the physical shape diagram, which appears on the colour liquid crystal display. When a target physical shape is selected, it is changed in brightness, or is flickered.

The person can select a desired physical shape in the physical shape diagram. If the selection cannot be made within a limited time, the "IDEAL HEALTH TYPE" at the centre of the physical shape diagram can be automatically selected.

Means 17 permits the person to select a desired frequency (number of times) and period for exercise among those given by a menu describing how often he takes a bodily exercise each unit time, for examples, once every day, once a week, three times a week, and how long he continues the scheduled exercise, for examples, three months, six months or one year, and set the number of times of the exercise as a target value.

No data of frequency and period are inputted within a given limited time, and then, assumedly appropriate frequency and period are automatically selected in consideration of the current physical shape and the target physical shape, and the so selected data is inputted, so that the number of times of bodily exercise required for attaining the target physical shape is automatically determined and given as a set value.

Means 18 compares the current physical shape with the target physical shape to determine the amount of bodily exercise per each exercise, which amount would be required for reducing the difference therebetween. The amount of bodily exercise (product of load and time) is given by:

$$\text{Amount of Bodily Exercise (kcal)} = \text{Exercise Hardness (kcal/min.)} \times \text{Exercise Period (min.)}$$

Amount of Bodily Exercise can be increased by increasing exercise hardness and/or exercise period.

In determining the required amount of bodily exercise first, the amount of fat to be consumed is determined from the difference between the current physical shape and the target physical shape per each exercise

Bodily fat of 1 kg is equivalent to 7000 kcal. The amount of fat to be consumed is expressed in terms of calorie amount, and the amount of exercise is assumed to be equivalent to the so expressed calorie amount. The calorie amount per unit time which can be consumed by taking a bodily exercise can be determined from the basic metabolism and the hardness efficiency at a selected level by following equation 3:

$$\text{Consumed Calorie Amount per Unit Time} = 2.35 \times \text{Hardness Efficiency} \times \text{Basic Metabolic Amount}/1440,$$

$$\text{where the Basic Metabolic Amount} = C_o \times \text{Weight} \times (100 - \text{Bodily Fat Rate})/100 + C_l$$

| | |
|---|---|
| Co = 24.0349 | female at the age of 40 or below |
| 21.951 | female above the age of 40 |
| 27.717 | male at the age of 40 or below |
| 25.333 | male above the age of 40 |
| Cl = 427.64 | female at the age of 40 or below |
| 424.38 | female above the age of 40 |
| 188.21 | male at the age of 40 or below |
| 243.28 | male above the age of 40 |

Hardness efficiency can be given for each level as follows:

| | |
|---|---|
| Level 1 | Hardness Efficiency = 0.825 |
| Level 2 | = 0.850 |
| Level 3 | = 0.875 |
| Level 4 | = 0.900 |
| Level 5 | = 0.925 |
| Level 6 | = 0.950 |
| Level 7 | = 0.975 |
| Level 8 | = 1.000 |
| Level 9 | = 1.025 |
| Level 10 | = 1.050 |
| Level 11 | = 1.075 |
| Level 12 | = 1.100 |
| Level 13 | = 1.125 |
| Level 14 | = 1.150 |

Hardness efficiency in bodily exercise is selected from the above by referring to which level is most in conformity with the one's exercising capability estimated by the means 14.

In case that no level is selected, a standard level is automatically selected.

Once the level has been manually or automatically set, the time required for burning and consuming extra amount of fat can be determined by dividing the amount of bodily exercise (expressed in terms of calorie) by the calorie per unit time consumed by the bodily exercise.

The length of period thus determined must be 12 minutes long or more; it is a minimum length of period required for starting the burning of bodily fat.

Means 19 compares the current physical shape with the target physical shape for each exercise to determine the difference therebetween in terms of calorie, thus informing the exercise amount determining means 18 of the difference as representing the exercise amount (expressed in terms of calorie) required for consuming the extra amount of fat. Then, the means 18 makes correction for the exercise amount, if necessary.

As may be understood from the above, persons can determine how much, how often and how long they should take bodily exercises to reach desired physical shape by referring to exercise programs shown on the colour liquid crystal display, permitting the variables once set to be corrected while taking bodily exercises. Thus, most effective, adaptive control can be made on bodily exercises.

Means 21 determines on the basis of the required amount of exercise provided by the means 18 and the number of times of exercise set by the means 17, an exercise program describing how long the aerobics machine is to be used each time of exercise, and how much heartbeat rate is set as target to fulfil the required amount of bodily exercise.

Means 22 compares the instantaneous heartbeat rate given by the means 20 with the one set for the required heartbeat rate, and in order to reduce the difference therebetween means 22 changes the amount of load on the load-variable aerobics machine by changing the gradient of the running belt B, the resistance due to the friction of the pedals P, the braking torque and other variables. Then, the actual heartbeat rate can be retained within the heartbeat zone within which the hardness of exercise (determined by the means 18 as appropriate) is retained.

Indirect type of aerobics machines permit the controlling of load in terms of the heartbeat rate as described above. Direct type of aerobics machines, however, attains the controlling of load irrespective of the heartbeat rate.

In the latter case it is necessary that calculations be made to determine how much the machine is loaded, and how long a selected bodily exercise is to be continued for each time of exercise by using the so loaded machine to fulfil the required amount of bodily exercise. Then, an exercise program describing how much the aerobics machine is loaded and how long the aerobics machine is to be used, is described. The block diagram of Fig.5 can be applied to both types of aerobics machines.

**Possibility of Industrial Utility**

As is apparent from the above, an automatic load-controlling apparatus for use in an aerobics machine according to the present invention can determine and provide, on the basis of a desired physical shape, an exercise program describing how much the machine is to be loaded, how often and how long the so loaded machine is to be used. Also, the automatic load-controlling apparatus can automatically handle the load controlling means of the aerobics machine so that the machine spontaneously follow the loading condition derived from the exercise program, thus assuring that persons are permitted to take bodily exercise for reaching the desired physical shape in a most adaptive, effective way without being disturbed by controlling the machine.

The automatic load-controlling apparatus permits persons to have the athletic type, the healthy type, the slender type and other specific physical shape which they can easily have in mind as a target for aerobics, and therefore, aerobics machines equipped with such automatic load-controlling apparatus will be most convenient to persons who take bodily exercises for the purpose of bodily shaping.

**Claims**

1. An automatic load control for a load-variable aerobics machine comprising:

   a bodily impedance measuring means having electrodes to be applied to a human body for determining the impedance of the human body;
   data input means for inputting individual data pertaining to sex, age, height and weight;
   means for determining the instantaneous fat rate from the body impedance and the individual data;
   means for determining the current physical shape from the weight and bodily fat rate;
   means for setting a desired physical shape as a target;
   means for determining the number of times (or frequency) of exercise and setting the so determined exercise amount for a set exercise amount value;
   means for comparing the current physical shape with the target physical shape to determine the amount of exercise required for reducing the difference therebetween;
   means for determining on the basis of the required amount of exercise and the set exercise amount value an exercise program describing how much the load-variable aerobics machine is to be loaded, and how long the so loaded aerobics machine is to be used; and
   means for changing the amount of load on the load-variable aerobics machine to be in conformity with the load given in the exercise program,

   whereby the loading of the aerobics machine may be controlled to consume the amount of exercise required for reaching the target physical shape according to the exercise program.

2. An automatic load control for a load-variable aerobics machine comprising:

   a bodily impedance measuring means having electrodes to be applied to a human body for determining the impedance of the human body;

data input means for inputting individual data pertaining to sex, age, height and body-weight;

means for determining the instantaneous fat rate from the bodily impedance and the individual data;

means for making a decision of the current physical shape from the bodily fat and the body-weight;

means for setting a desired physical shape for a target;

means for determining the number of times of exercise and for setting the so determined number of times of exercise as a target;

means for comparing the current physical shape with the target physical shape to determine the amount of exercise required for reducing the difference therebetween;

means for determining on the basis of the required exercise amount and the target exercise amount an exercise program describing a target heartbeat rate and a length of period for using the aerobics machine;

means for measuring the heartbeat rate during exercise; and

means for changing the amount of load on the load-variable aerobics machine to permit the heartbeat rate measured during exercise to be equal to the target heartbeat rate,

whereby the loading of the aerobics machine may be controlled to consume the amount of exercise required for reaching the target physical shape according to the exercise program.

FIG. 1

FIG. 2

F I G. 3

# F I G . 4

load/target
        heartbeat rate

length of period for using
the aerobics machine

FIG. 6

H

HL                    HR

H1        H2        H2        H1

F I G. 7

EP 1 075 858 A1

16

# F I G. 8

Ideal Bodyweight=(Height-100)×0.9×0.91

F I G. 9

THE CURRENT PHYSICAL SHAPE

THE TARGET PHYSICAL SHAPE

# INTERNATIONAL SEARCH REPORT

International application No.

PCT/JP99/02245

| A. CLASSIFICATION OF SUBJECT MATTER |
|---|
| Int.Cl⁶ A63B22/06, 23/04, 71/06, A61B5/05 |

According to International Patent Classification (IPC) or to both national classification and IPC

| B. FIELDS SEARCHED |
|---|
| Minimum documentation searched (classification system followed by classification symbols) |
| Int.Cl⁶ A63B22/06, 23/04, 71/06, A61B5/05 |

Documentation searched other than minimum documentation to the extent that such documents are included in the fields searched

| Jitsuyo Shinan Koho | 1922–1996 | Toroku Jitsuyo Shinan Koho | 1994–1999 |
|---|---|---|---|
| Kokai Jitsuyo Shinan Koho | 1971–1999 | Jitsuyo Shinan Toroku Koho | 1996–1999 |

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
|---|---|---|
| Y | JP, 6-36839, Y2 (YAMAN Ltd.), 28 September, 1994 (28. 09. 94), Full text ; Figs. 1 to 5 (Family: none) | 1, 2 |
| Y | JP, 2534464, B2 (YAMAN Ltd.), 18 September, 1996 (18. 09. 96), Full text ; Figs. 1 to 9 (Family: none) | 1, 2 |
| Y | JP, 8-126632, A (Omron Corp.), 21 May, 1996 (21. 05. 96), Full text ; Figs. 1 to 12 (Family: none) | 1, 2 |
| Y | JP, 8-280840, A (Matsushita Electric Works,Ltd.), 29 October, 1996 (29. 10. 96), Full text ; Figs. 1 to 9 | 2 |
| A | Full text ; Figs. 1 to 9 (Family: none) | 1 |

☐ Further documents are listed in the continuation of Box C.   ☐ See patent family annex.

| * Special categories of cited documents: | "T" later document published after the international filing date or priority |
|---|---|
| "A" document defining the general state of the art which is not considered to be of particular relevance | date and not in conflict with the application but cited to understand the principle or theory underlying the invention |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| "O" document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" document published prior to the international filing date but later than the priority date claimed | "&" document member of the same patent family |

| Date of the actual completion of the international search | Date of mailing of the international search report |
|---|---|
| 22 July, 1999 (22. 07. 99) | 3 August, 1999 (03. 08. 99) |

| Name and mailing address of the ISA/ Japanese Patent Office | Authorized officer |
|---|---|
| Facsimile No. | Telephone No. |

Form PCT/ISA/210 (second sheet) (July 1992)